# EUROPÄISCHE PATENTSCHRIFT

(11) **EP 1 281 360 B1**
(45) Veröffentlichungstag und Bekanntmachung des Hinweises auf die Patenterteilung: **02.03.2005**
(21) Anmeldenummer: 02016154.3
(22) Anmeldetag: 20.07.2002
(51) Int. Cl.: A61B 17/34, A61F 2/00, A61B 17/32

(54) **Medizinisches Instrument**
Surgical instrument for endoscopic use
Instrument chirurgical endoscopique

(30) Priorität: 03.08.2001 AT 12152001
(43) Veröffentlichungstag der Anmeldung: 05.02.2003
(73) Patentinhaber: AMI Agency for Medical Innovations GmbH, 6840 Götzis (AT)
(72) Erfinder: Egle, Walter, 6842 Koblach (AT)
(74) Vertreter: Hefel, Herbert, Dipl.-Ing.

(56) Entgegenhaltungen:
- EP-A- 1 010 395
- WO-A-01/95810
- US-A- 5 037 433
- US-A- 5 817 111
- US-A- 5 908 381
- US-A- 5 910 149

## Beschreibung

Die Erfindung betrifft ein medizinisches Instrument mit einem länglichen Körper, der einen zum distalen Ende des Körpers hin offenen Aufnahmeraum für ein chirurgisches Implantat aufweist

Zum Einbringen von chirurgischen Implantaten in Körperhöhlen werden heute in der minimalinvasiven Chirurgie häufig Trokare verwendet. Solche Trokare werden beispielsweise bei laparoskopischen und thoraskopischen Eingriffen eingesetzt. Durch die von außerhalb des Körpers in den Operationsraum führenden hülsenförmigen Trokaren können Instrumente und Gegenstände in die Körperhöhle eingeführt werden. Bei einem laparoskopischen Eingriff wird üblicherweise vor dem Eingriff ein Gas eingeblasen, wobei die Trokare Ventile aufweisen und bei einer Operation durch die Trokare hindurch das Pneumoperitoneum aufrechterhalten bleibt.

Soll ein medizinisches Implantat in die Körperhöhle eingebracht werden, so ist dessen völlige Sterilität zu gewährleisten, es darf also beispielsweise nicht mit der Haut des Patienten in Berührung kommen. Bei der Einführung des sterilisierten Implantats in die Körperhöhle durch ein Trokar mittels eines medizinischen Instrumentes, herkömmlicherweise einer Fasszange, ist somit äußerste Sorgfalt anzuwenden. Die Präparation der Stelle, an der das Implantat anzubringen ist, erfolgt üblicherweise durch ein oder mehrere weitere Trokare, ebenso wie andere operative Schritte, beispielsweise Saug-Spülvorgänge oder elektrochirurgische Maßnahmen üblicherweise durch weitere Trokare erfolgen.

Aus der US-PS 5,171,240 A ist ein medizinisches Instrument der eingangs genannten Art bekannt. Der Aufnahmeraum des Instruments zum Einsetzen eines Protheseteils in einem Ohr weist hier einen vom offenen distalen Ende ausgehenden sich verjüngenden Schlitz auf, der zum Einklemmen des einzusetzenden Teils dient. Dieses Instrument stellt somit eine Art Halteinstrument dar und ersetzt eine Zange zum Greifen des Implantats.

Ein weiteres medizinisches Instrument zum Einbringen eines Implantats in Form eines netzartigen Teils ist aus der EP 0 581 036 A1 bekannt. Das Implantat ist durch einen seitlichen Schlitz in den Aufnahmeraum einführbar und wird um den Schaft einer Haltezange gewickelt.

Die US-PS 5,400,773 A zeigt weiters ein medizinisches Instrument mit einem aufblasbaren endoskopischen Retraktor als Arbeitsgerät, welcher einen Kanal des Instruments durchsetzt. Ein zweites medizinisches Arbeitsgerät durchsetzt einen weiteren Kanal des Instruments.

Die EP 1010395 zeigt ein medizinisches Instrument mit einem länglichen Körper und einem durchgehenden Kanal, durch den sich ein medizinisches Arbeitsgerät erstreckt.

Aufgabe der Erfindung ist es, ein medizinisches Instrument bereitzustellen, durch welches das Einführen des Implantats durch ein Trokar sowie dessen operative Anbringung im menschlichen Körper erleichtert wird. Erfindungsgemäß gelingt dies durch ein medizinisches Instrument mit den Merkmalen des Anspruchs 1.

Das chirurgische Implantat wird in sterilisierter Form in den Aufnahmeraum des Körpers des Instruments eingebracht. Gemäß der Erfindung erfolgt die Auslieferung des Instruments an ein Krankenhaus bereits mit dem darin eingebrachten lmplantat, zu welchem Zweck das Instrument mit dem darin eingebrachten Implantat steril verpackt ist. Die distale Öffnung des Aufnahmeraums kann dabei, falls erforderlich, entsprechend verschlossen sein, beispielsweise mit einem sterilen Klebeband. Das erfindungsgemäße Instrument umfaßt außerdem ein Arbeitsgerät zur Durchführung eines bei der Implantation erforderlichen Operationsschrittes. Beispielsweise kann es sich hierbei um einen Präparationsschritt für eine bei der Implantation des Implantats erforderliche Präparation des Körpergewebes handeln. Es kann dadurch die Implantation vereinfacht werden. Weiters kann ein andernfalls für ein separates Arbeitswerkzeug erforderliches Trokar eingespart werden.

Anstelle eines Präparationsinstrumentes kann das medizinische Arbeitsgerät beispielsweise auch von einem Saug-Spülgerät, von einer Fasszange, einer Schere, einem Clip-Instrument oder einem elektrochirurgischen Instrument gebildet werden. Jeweils kann mit dem Arbeitsgerät ein bei der Implantation erforderlicher Arbeitsvorgang ausgeführt werden.

In einer vorteilhaften Ausführungsform der Erfindung ist das medizinische Instrument zum Einbringen eines sogenannten Magenbandes aus Silikon vorgesehen.

Solche Magenbänder werden insbesondere bei Operationen gegen Fettleibigkeit um den Magen gelegt. Ein medizinisches Instrument kann auch für andere nichtautologe chirurgische Implantate zum Einbringen in menschliche oder tierische Körperhöhlen vorgesehen sein, beispielsweise für Gefäßprothesen. Solche Gefäßprothesen sind u.a. Y-Gefäßprothesen, wie sie für die Bauchaorta vorgesehen sind. Die einzubringenden nicht-autologen Implantate können aus Kunststoff, Metall oder anderen für Implantate geeigneten Materialien bestehen.

Weitere Vorteile und Einzelheiten der Erfindung werden im folgenden anhand der in den Zeichnungen dargestellten Ausführungsbeispiele erläutert. In der Zeichnung zeigen:
- Fig. 1: eine Seitenansicht eines ersten Ausführungsbeispiels eines erfindungsgemäßen Instruments;
- Fig. 2: einen Schnitt entlang der Linie A-A von Fig. 1;
- Fig. 3: ein Detail Z von Fig. 2;
- Fig. 4: den distalen Bereich des Instruments in einem Fig. 2 entsprechenden Schnitt mit ausgefahrener Präparierspitze;
- Fig. 5: das distale Ende einer etwas modifizierten Ausführungsform in einer perspektivischen Darstellung;
- die Fig. 6 und 7: schematische Seitenansichten von weiteren Ausführungsformen der Erfindung;
- die Fig. 8: ein vergrößertes Detail Y der Fig. 7;
- die Fig. 9 und 10: weitere Ausführungsformen der Erfindung;
- die Fig. 11 und 12: schematische Darstellungen der distalen Enden von weiteren Ausführungsformen erfindungsgemäßer medizinischer Instrumente.

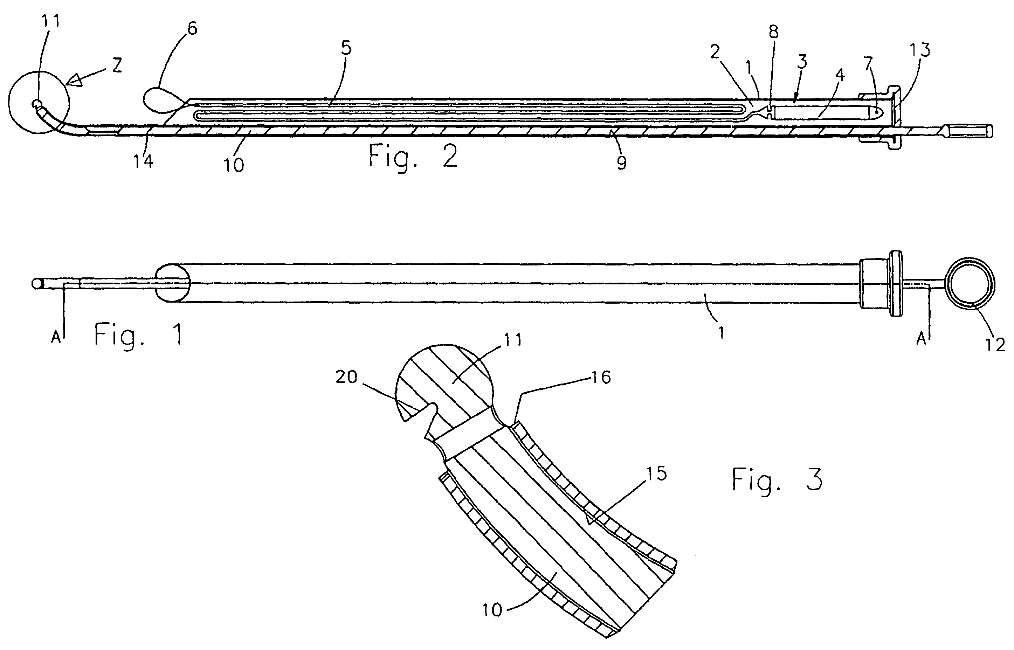

Die Fig. 1 bis 4 zeigen eine erste Ausführungsform der Erfindung. Das erfindungsgemäße medizinische Instrument weist einen länglichen Körper 1 mit einer zylinderförmigen Mantelfläche auf. Innerhalb dieses Körpers 1 ist ein zum distalen Ende des Instruments hin offener Aufnahmeraum 2 für ein chirurgisches Implantat 3 vorgesehen. Beim gezeigten Ausführungsbeispiel handelt es sich dabei um ein sogenanntes Magenband, das um die Magenwand distal zum Sphinctercardia zu legen ist. Das Magenband besteht aus einem einen zentralen Hohlraum aufweisenden Band aus Silikon 4 (wobei eine textile Netzeinlage eine Längenänderung des Bandes 4 verhindert) und einem daran angeschlossenen dünnen Schlauch 5, der an seinem Ende mit einer Schlinge 6 versehen ist. Am anderen Ende des Bandes 4 ist eine Öse 7 vorgesehen, durch die der auf der gegenüberliegenden Seite liegende Abschnitt des Bandes gezogen werden kann, wobei eine nutartige Vertiefung 8 zum Einrasten in die Öse 7 und Verschluß des um das Körperorgan gelegten Bandes 4 dient. Durch den Schlauch 5 kann eine Salzlösung eingebracht werden, so daß die innere Öffnung des um das Körperorgan gelegten Bandes eingestellt werden kann. Solche Magenbänder sind bekannt.

Das Instrument weist weiters eine in Längsrichtung des Körpers 1 des Instruments sich erstreckenden, durchgehenden Kanal 9 auf, durch den sich ein medizinisches Arbeitsgerät 10 erstreckt. Der Aufnahmeraum 2 und der durchgehende Kanal 9 sind dabei durch eine Zwischenwand im Körper 1 voneinander getrennt. Das medizinische Arbeitsgerät 10 weist als Arbeitskopf 11 eine am Ende stumpf (atraumatisch) ausgebildete Präparierspitze auf, und zwar am distalen Ende des Arbeitsgerätes, sowie an seinem proximalen Ende einen Betätigungsgriff 12, der hier als Öse ausgebildet ist. Das Arbeitsgerät 10 ist weiters in Längsrichtung des Körpers 1 des Instruments mittels des Betätigungsgriffes 12 verschiebbar.

Am proximalen Ende wird der Körper 1 des Instruments durch eine Kappe 13 verschlossen, durch welche der Aufnahmeraum 2 sowie der Kanal 9 zum proximalen Ende des Instruments hin gasdicht verschlossen werden. In der Kappe 13 ist eine Bohrung vorgesehen, durch welche das Arbeitsgerät 10 abgedichtet durchtritt. Hierzu kann beispielsweise die Kappe 13 aus Silikon ausgebildet sein und dicht an der in diesem Teil zylindrisch ausgebildeten Mantelfläche des Arbeitsgerätes 10 anliegen. Es könnte andererseits oder zusätzlich auch eine Dichtung zwischen der Innenwandung des Kanals 9 und der Mantelfläche des Arbeitsgerätes 10, beispielsweise in einem mittleren Bereich des Instruments vorgesehen sein.

Am distalen Ende des Körpers 1 erstreckt sich ein Fortsatz 14 mit einer in Längsrichtung des Fortsatzes 14 verlaufenden, durchgehenden Öffnung 15. Beispielsweise wird dieser Fortsatz 14 von einem Röhrchen gebildet, das innerhalb des Körpers 1 bis zum proximalen Ende desselben verläuft und auf diese Weise auch den Kanal 9 durch den Körper 1 sowie die Zwischenwandung zwischen dem Aufnahmeraum 2 und dem Kanal 9 bildet. Die Öffnung 15 im Fortsatz 14 verlängert somit den Kanal 9 und der Fortsatz 14 im Bereich seines distalen Endes kreisbogenförmig gekrümmt ausgebildet. Das durch die Öffnung 15 des Fortsatzes 14 verlaufende Arbeitsgerät 10 ist in diesem Bereich entsprechend der durch den Fortsatz 14 verlaufenden Öffnung 15 gekrümmt.

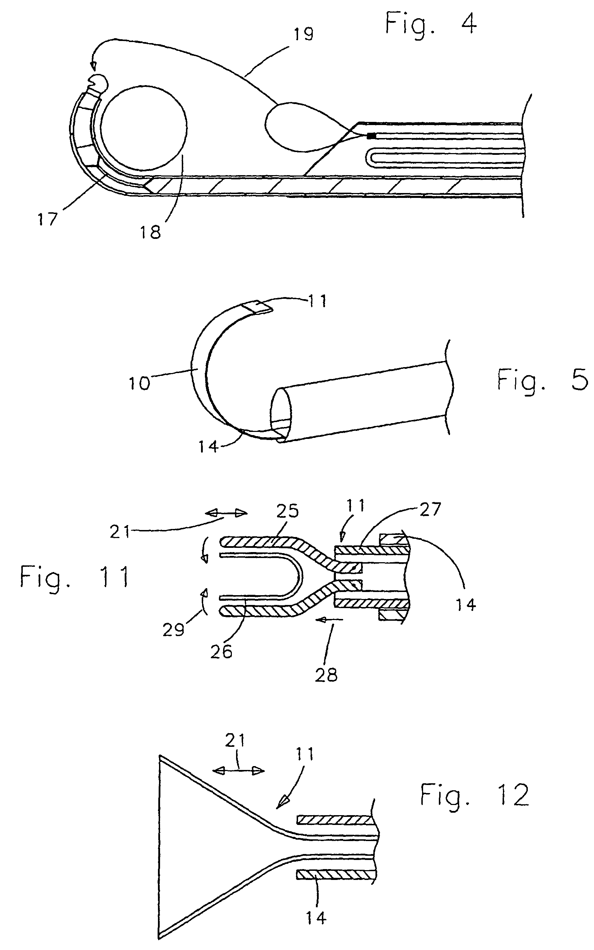

Das Arbeitsgerät 10 ist durch Verschieben des Betätigungsgriffes 12 in Längsrichtung des Körpers 1 gegenüber dem Körper 1 bzw. dem daran angebrachten Fortsatz 14 verschiebbar. In der in den Fig. 1 bis 3 dargestellten zurückgezogenen Position des Arbeitsgerätes 10 liegt der Arbeitskopf 11 dem distalen Ende 16 des Fortsatzes 14 benachbart. In Fig. 4 ist die vorgeschobene Stellung des Arbeitsgerätes dargestellt. Das Arbeitsgerät 10 ragt hier weiter aus dem distalen Ende 16 des Fortsatzes heraus, wobei es die endseitige kreisbogenförmige Krümmung dieses Fortsatzes fortsetzt. Die dabei erforderliche Krümmung des Arbeitsgerätes 10 wird hierbei von einem abgeflachten, elastisch biegbaren Abschnitt 17 aufgenommen. Im Bereich zwischen dem Arbeitskopf 11 und dem Betätigungsgriff 12 weist das Arbeitsgerät 10 ansonsten einen kreisrunden Querschnitt auf.

In Fig. 4 ist weiters schematisch der distale Ösophagus 18 eingezeichnet, um den das Magenband herumzulegen ist. Mittels dem als Präparierinstrument ausgebildeten Arbeitsgerät 10 wird der für das Herumziehen des Magenbandes erforderliche Raum beschaffen, indem das den Ösophagus umgebende Körpergewebe mit dem als Präparierspitze ausgebildeten Arbeitskopf 11 beiseite gedrückt wird. Dieser Vorgang erfolgt zunächst bei zurückgezogenem Betätigungsgriff (Fig. 1 bis 3). In der Folge wird der Betätigungsgriff langsam in seine vordere Position gebracht, so daß die in Fig. 4 dargestellte Stellung des Arbeitsgerätes 10 erreicht wird. Das erfindungsgemäße medizinische Instrument ist dabei durch einen in den Bauchraum gesetzten Trokar in diesen eingeführt. Aufgrund der Schleusenfunktion des Trokars und der gasdichten Abdichtung des Aufnahmeraumes 2 und des Arbeitsgerätes 10 gegenüber dem Kanal 9 des medizinischen Instruments wird das Pneumoperitoneum dabei aufrechterhalten. In der Folge wird mit einem medizinischen Greifinstrument, das durch einen weiteren Trokar in die Bauchhöhle ragt, die Schlinge 6 gefaßt und unter Herausziehen des anschließenden Bereiches des Schlauches 5 aus dem Aufnahmeraum 2 über den Arbeitskopf 11 gelegt, wie dies symbolisch durch den Pfeil 19 in Fig. 4 angedeutet ist. Die Schlinge 6 wird dabei in den im Arbeitskopf 11 vorgesehenen Schlitz 20 eingehängt, der eine Halterungseinrichtung zur Befestigung der Schlinge 6 bildet. In der Folge wird der Betätigungsgriff 12 langsam zurückgezogen, bis er die in den Fig. 1 bis 3 dargestellte Position erreicht hat. Anschließend wird das gesamte Instrument zurückgezogen. Mit dem durch einen anderen Trokar eingeführten Greifinstrument kann dabei das Herausziehen des Schlauches und in der weiteren Folge des Bandes 4 unterstützt werden. Die Schlinge 6 wird dann aus dem Schlitz 20 des Arbeitskopfes 11 herausgezogen und in der üblichen Operationstechnik wird der Schlauch 5 durch die Öse 7 gezogen, bis die Vertiefung 8 in dieser liegt und der Schlauch entsprechend um das Körperorgan angebracht ist.

Die Halterungseinrichtung im Arbeitskopf 11 könnte auch könnte auch als Stufe, Klemmteil usw. ausgebildet sein. In einer etwas modifizierten Ausführungsform könnte das freie Ende des Schlauches 5 auch von vorneherein an einer am Arbeitskopf 11 vorgesehenen Halterungseinrichtung befestigt sein, so daß der Schlauch 5 bereits beim Präparationsvorgang um das Körperorgan mitgeführt wird. In der in Fig. 4 dargestellten Stellung des Arbeitsgerätes 10 könnte der Schlauch 5 dann mittels eines weiteren medizinischen Greifinstrumentes aus der Halterungseinrichtung am Arbeitskopf 11 herausgezogen und vollständig um das Körperorgan gezogen werden.

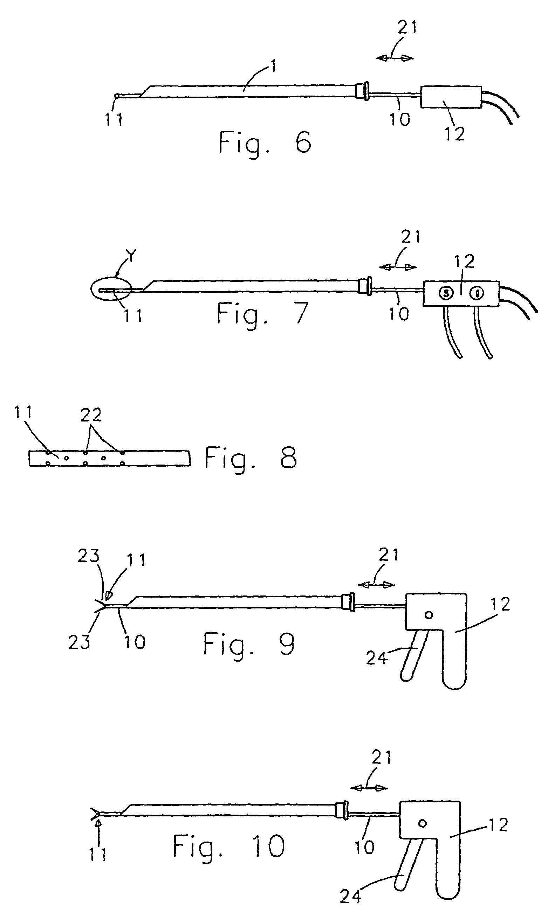

Bei dem in Fig. 5 dargestellten etwas modifizierten Ausführungsbeispiel ist der Kanal im Körper 1 und die diesen Kanal fortsetzende Öffnung im Fortsatz 14 im Querschnitt als flaches Rechteck ausgebildet und das Arbeitsgerät 10 wird zumindest in seinem an den Arbeitskopf 11 anschließenden Bereich von einem gekrümmten Federstahl gebildet. Wenn der Arbeitskopf 11 somit in die in Fig. 5 dargestellte Position vorgeschoben wird, krümmt sich dieser Federstahl des Arbeitsgerätes in seine vorgespannte Position, so daß die gezeigte Krümmung erreicht wird. Anstelle eines Federstahls könnte auch ein anderes entsprechend elastisch verformbares Material vorgesehen sein, beispielsweise ein Kunststoffmaterial.

Bei dem in Fig. 6 dargestellten Ausführungsbeispiel ist als Arbeitsgerät ein elektrochirurgisches Instrument vorgesehen. Der Arbeitskopf 11 stellt hier einen Koagulations-Ball zum Veröden eines Gefäßes dar. Durch einen am Betätigungsgriff 12 vorgesehenen Taster kann an den Arbeitskopf 11 eine Spannung gegenüber dem Körpergewebe des Patienten angelegt werden. Das Arbeitsgerät 10 ist hierbei wiederum gegenüber dem Körper 1 in Längsrichtung des Körpers 1 verschiebbar, wie durch den Pfeil 21 angedeutet ist.

Beim Ausführungsbeispiel gemäß den Figuren 7 und 8 ist das Arbeitsgerät als Saug-Spülgerät ausgebildet, wobei durch Betätigen von entsprechenden Tastern eine Spülflüssigkeit aus dem Arbeitskopf 11 durch Öffnungen 22 auspumpbar bzw. Flüssigkeit aufsaugbar ist.

Beim Ausführungsbeispiel gemäß Fig. 9 ist das Arbeitsgerät 10 als Greifinstrument ausgeführt, wobei Greifarme 23 mittels eines Schwenkhebels 24 am Betätigungsgriff 12 aneinander anlegbar oder auseinanderklappbar sind.

Beim Ausführungsbeispiel gemäß Fig. 10 weist das Arbeitsgerät 10 als Arbeitskopf 11 eine Schere auf, die durch Verschwenken des Schwenkhebels 24 am Betätigungsgriff 12 betätigbar ist.

Bei dem in Fig. 11 dargestellten Ausführungsbeispiel ist das Arbeitsgerät als Clip-Instrument ausgebildet. Am Arbeitskopf 11 sind Klemmarme 25 vorgesehen, zwischen denen ein Clipteil 26 gelagert ist. Durch Verschieben der Hülse 27 in Richtung des Pfeils 28 werden die Klemmarme 25 gegeneinander verschwenkt (Pfeil 29), wobei das Clipteil 26 zusammengedrückt wird.

Bei dem in Fig. 12 dargestellten Ausführungsbeispiel ist das Arbeitsgerät als Retraktor ausgebildet. In der gegenüber dem Fortsatz 14 zurückgezogenen Position ist der Arbeitskopf 11 zusammengeklappt. In seiner vorgeschobenen Position ist er fächerförmig auseinandergefaltet und kann zum Fernhalten von Körpergewebe von der Operationsstelle eingesetzt werden. Solche Retraktoren werden beispielsweise bei der Implantation von Gefäßprothesen eingesetzt.

Die Arbeitsgeräte dieser in den Fig. 6 bis 12 dargestellten Ausführungsformen sind jeweils relativ zum Körper 1 des Instruments verschiebbar, wie dies durch die Doppelpfeile 21 angedeutet ist. Im Zusammenhang mit einem erfindungsgemäßen Instrument können unterschiedliche solche Arbeitsgeräte eingesetzt werden, wie sie von ihrer Funktion her ebenso wie in ihrem grundsätzlichen Aufbau hinlänglich bekannt sind.

Durch ein erfindungsgemäßes Instrument kann ein Implantat ohne Berührung durch den Chirurgen bzw. die OP-Schwester direkt in die betreffende Körperhöhle des Menschen oder Tieres eingebracht werden. Weiters kann durch das medizinische Instrument bei der Implantation des Implantats mittels des Arbeitsgerätes eine bestimmte Funktion ausgeführt werden, z.B. der für das Implantat notwendige Raum geschaffen werden bzw. die notwendige chirurgische Präparation dieser Stelle durchgeführt werden. Weiters dient ein erfindungsgemäßes Instrument zur sicheren Verpackung eines Implantats für den Transport desselben sowie für die Gewährleistung von seiner Sterilität.

### Legende

### zu den Hinweisziffern:

- 1: Körper
- 2: Aufnahmeraum
- 3: Implantat
- 4: Band
- 5: Schlauch
- 6: Schlinge
- 7: Öse
- 8: Vertiefung
- 9: Öffnung
- 10: Arbeitsgerät
- 11: Arbeitskopf
- 12: Betätigungsgriff
- 13: Kappe
- 14: Fortsatz
- 15: Öffnung
- 16: distales Ende
- 17: Abschnitt
- 18: Ösophagus
- 19: Pfeil
- 20: Schlitz
- 21: Pfeil
- 22: Öffnung
- 23: Greifarm
- 24: Schwenkhebel
- 25: Klemmarm
- 26: Clipteil
- 27: Hülse
- 28: Pfeil
- 29: Pfeil

## Patentansprüche

1. Medizinisches Instrument mit einem länglichen Körper (1), der einen in Längsrichtung des Körpers (1) sich erstreckenden, durchgehenden Kanal (9) aufweist , durch den sich ein medizinisches Arbeitsgerät (10) erstreckt, welches gegenüber dem Körper (1) in Längsrichtung des Körpers (1) verschiebbar ist und mit seinen distalen und proximalen Enden aus dem durchgehenden Kanal (9) herausragt, wobei es im Bereich seines distalen Endes einen Arbeitskopf (11) und im Bereich seines proximalen Endes einen Betätigungsgriff (12) aufweist, **dadurch gekennzeichnet, daß** der längliche Körper (1) des medizinischen Instruments zusätzlich zum durchgehenden Kanal (9) einen zum distalen Ende des Körpers hin offenen Aufnahmeraum (2) aufweist, in den ein chirurgisches Implantat (3) eingebracht ist, und daß das medinische Instrument mit dem darin eingebrachten Implantat (3) steril verpackt ist, wobei mittels des medizinischen Instruments das Implantat (3) berührungsfrei in eine Körperhöhle eines Menschen oder Tieres einbringbar ist und bei der Implantation des lmplantats (3) mit dem Arbeitsgerät (10) des medizinischen Instruments ein Operationsschritt durchführbar ist.

2. Medizinisches Instrument nach Anspruch 1, **dadurch gekennzeichnet, daß** der Aufnahmeraum (2) des Körpers (1) nur zum distalen Ende des Körpers (1) hin offen ist.

3. Medizinisches Instrument nach Anspruch 2, **dadurch gekennzeichnet, daß** zum Verschluß des proximalen Endes des Aufnahmeraums (2) eine das proximale Ende des Körpers (1) überdeckende Kappe (13) vorgesehen ist, die eine Bohrung zum Durchtritt des Arbeitsgerätes (10) aufweist.

4. Medizinisches Instrument nach einem der Ansprüche 1 bis 3, **dadurch gekennzeichnet, daß** die Mantefläche des Arbeitsgerätes (10) gegenüber der Seitenwand des durchgehenden Kanals (9) des Körpers (1) oder gegenüber einer Bohrung in einer das proximale Ende des Körpers (1) überdeckenden Kappe (13), durch welche das Arbeitsgerät (10) durchtritt, abgedichtet ist.

5. Medizinisches Instrument nach einem der Ansprüche 1 bis 4, **dadurch gekennzeichnet, daß** die Mantelfläche des Körpers (1) zylindermantelförmig ausgebildet ist.

6. Medizinisches Instrument nach einem der Ansprüche 1 bis 5, **dadurch gekennzeichnet, daß** das vom Aufnahmeraum (2) aufzunehmende Implantat ein Magenband ist.

7. Medizinisches Instrument nach einem der Ansprüche 1 bis 6, **dadurch gekennzeichnet, daß** der Arbeitskopf (11) des Arbeitsgerätes (10) eine an ihrem freien Ende stumpf ausgebildete Präparierspitze ist.

8. Medizinisches Instrument nach Anspruch 7, **dadurch gekennzeichnet, daß** in der Präparierspitze eine Halterungseinrichtung zur Befestigung einer Schlinge (6) des Implantats vorgesehen ist.

9. Medizinisches Instrument nach Anspruch 8, **dadurch gekennzeichnet, daß** die Halterungseinrichtung ein Schlitz (20) in der Präparierspitze ist.

10. Medizinisches Instrument nach einem der Ansprüche 1 bis 9, **dadurch gekennzeichnet, daß** am distalen Ende des Körpers (1) ein Fortsatz (14) mit einer in Längsrichtung des Fortsatzes (14) verlaufenden durchgehenden Öffnung (15) angeordnet ist, welche die durch den Körper (1) durchgehenden Kanal (9) verlängert und durch welche sich das Arbeitsgerät (10) erstreckt, wobei der Fortsatz (14) und die durch ihn verlaufende Öffnung (15) zumindest im an das distale Ende (16) des Fortsatzes (14) anschließenden Bereich gekrümmt ausgebildet sind.

11. Medizinisches Instrument nach Anspruch 9, **dadurch gekennzeichnet, daß** die Krümmung in Richtung zum Aufnahmeraum (2) des Körpers (1) gerichtet ist.

12. Medizinisches Instrument nach einem der Ansprüche 1 bis 11, **dadurch gekennzeichnet, daß** das medizinische Arbeitsgerät (10) eine Fasszange oder eine Schere ist.

13. Medizinisches Instrument nach einem der Ansprüche 1 bis 11, **dadurch gekennzeichnet, daß** das medizinische Arbeitsgerät (10) ein Saug- und/oder Spülinstrument ist.

14. Medizinisches Instrument nach einem der Ansprüche 1 bis 11, **dadurch gekennzeichnet, daß** das medizinische Arbeitsgerät (10) ein elektrochirurgisches Instrument ist.

15. Medizinisches Instrument nach einem der Ansprüche 1 bis 11, **dadurch gekennzeichnet, daß** das medizinische Arbeitsgerät (10) ein Retraktor ist.

16. Medizinisches Instrument nach einem der Ansprüche 1 bis 11, **dadurch gekennzeichnet, daß** das medizinische Arbeitsgerät (10) ein Clip-Instrument ist.

17. Medizinisches Instrument nach einem der Ansprüche 1 bis 16, **dadurch gekennzeichnet, daß** der Aufnahmeraum (2) und der durchgehende Kanal (9) des Körpers (1) durch eine Zwischenwand innerhalb des Körpers (1) voneinander getrennt sind.

## Claims

1. A medical instrument having an elongated body (1) with a through-canal (9) extending in the longitudinal direction of the body (1), via which canal a medical operating instrument (10) extends which is displaceable in the longitudinal direction of the body (1) and projects from the through-canal (9) with its distal and proximal ends, the said operating instrument having an operating head (11) in the region of its distal end and an actuating grip (12) in the region of its proximal end, **characterised in that** the longitudinal body (1) of the medical instrument has, in addition to the through-canal (9), an accommodating chamber (2) open towards the distal end of the body, into which chamber a surgical implant (3) is introduced, and **in that** the medical instrument is sterile-packed with the implant (3) introduced therein, wherein the implant (3) is contactlessly introducible into a body cavity of a human or animal with the use of the medical instrument and, during implantation of the implant (3), a surgical step is performable with the operating instrument (10) of the medical instrument.

2. A medical instrument according to Claim 1, **characterised in that** the accommodating chamber (2) of the body (1) is open only towards the distal end of the body (1).

3. A medical instrument according to Claim 2, **characterised in that**, to close the proximal end of the accommodating chamber (2), a cap (13) covering the proximal end of the body (1) is provided, which cap has a bore for passage of the operating instrument (10).

4. A medical instrument according to any one of Claims 1 to 3, **characterised in that** the outer surface of the operating instrument (10) is sealed in respect of the side wall of the through-canal (9) of the body (1) or in respect of a bore in a cap (13) covering the proximal end of the body (1), through which cap the operating instrument (10) passes.

5. A medical instrument according to any one of Claims I to 4, **characterised in that** the outer surface of the body (1) is designed in the form of a cylindrical surface.

6. A medical instrument according to any one of Claims 1 to 5, **characterised in that** the implant to be accommodated by the accommodating chamber (2) is a gastric band.

7. A medical instrument according to any one of Claims 1 to 6, **characterised in that** the operating head (11) of the operating instrument (10) has a dissecting tip, which is of a blunt design at its free end.

8. A medical instrument according to Claim 7, **characterised in that** a retaining device is provided in the dissecting tip to attach a loop (6) of the implant.

9. A medical instrument according to Claim 8, **characterised in that** the retaining device is a slit (20) in the dissecting tip.

10. A medical instrument according to any one of Claims 1 to 9, **characterised in that** an extension (14) having a through-opening (15) extending in the longitudinal direction of the extension (14) is arranged at the distal end of the body (1), which through-opening prolongs the channel (9) passing through the body (1) and through which opening the operating instrument (10) extends, wherein the extension (14) and the opening (15) extending therethrough are curved in shape at least in the region adjoining the distal end (16) of the extension (14).

11. A medical instrument according to Claim 9, **characterised in that** the curvature is directed in the direction of the accommodating chamber (2) of the body (1).

12. A medical instrument according to any one of Claims 1 to 11, **characterised in that** the medical operating instrument (10) is gripping forceps or scissors.

13. A medical instrument according to any one of Claims to 11, **characterised in that** the medical instrument (10) is a suction and/or irrigation instrument.

14. A medical instrument according to any one of Claims to 11, **characterised in that** the medical instrument (10) is an electrosurgical instrument.

15. A medical instrument according to any one of Claims to 11, **characterised in that** the medical instrument (10) is a retractor.

16. A medical instrument according to any one of Claims to 11, **characterised in that** the medical instrument (10) is a clip instrument.

17. A medical instrument according to any one of Claims to seem, **characterised in that** the accommodating chamber (2) and the through-canal (9) of the body (1) are separated from one another by a partition wall within the body (1).

## Revendications

1. Instrument médical comprenant un corps allongé (1) qui présente un canal traversant (9) dans la direction longitudinale du corps (1) et dans lequel s'étend un outil médical (10) qui peut coulisser par rapport au corps (1) dans la direction longitudinale du corps (1) et émerge du canal traversant (9) à ses extrémités distale et proximale, cet outil présentant une tête de travail (11) dans la région de son extrémité distale et une poignée d'actionnement (12) dans la région de son extrémité proximale,
**caractérisé en ce que**
le corps allongé (1) de l'instrument médical présente, en supplément du canal traversant (9), une chambre de réception (2) qui s'ouvre vers l'extrémité distale du corps et dans laquelle un implant chirurgical (3) est mis en place, et l'instrument médical, avec l'implant (3) mis en place à l'intérieur, est empaqueté de façon stérile, de sorte qu'au moyen de l'instrument médical, l'implant (3) peut être mis en place sans contact dans une cavité corporelle d'un homme ou d'un animal et une phase d'une opération peut être exécutée avec l'outil (10) de l'instrument médical au cours de l'implantation de l'implant (3).

2. Instrument médical selon la revendication 1,
**caractérisé en ce que**
la chambre de réception (2) du corps (1) s'ouvre seulement sur l'extrémité distale du corps (1).

3. Instrument médical selon la revendication 2,
**caractérisé en ce que**
pour obturer l'extrémité proximale de la chambre de réception (2), il est prévu un capuchon (13) qui recouvre l'extrémité proximale du corps (1) et qui présente un perçage pour donner passage à l'outil (10).

4. Instrument médical selon une des revendications 1 à 3,
**caractérisé en ce que**
la surface latérale de l'outil (10) forme un joint étanche avec rapport à la paroi latérale du canal traversant (9) du corps (1) ou avec un perçage ménagé dans un capuchon (13) qui recouvre l'extrémité proximale du corps (1) et à travers lequel passe l'outil (10).

5. Instrument médical selon une des revendications 1 à 4,
**caractérisé en ce que**
la surface latérale du corps (1) a la forme d'une paroi de cylindre.

6. Instrument médical selon une des revendications 1 à 5,
**caractérisé en ce que**
l'implant qui doit être placé dans la chambre de réception (2) est un cerclage stomacal.

7. Instrument médical selon une des revendications 1 à 6,
**caractérisé en ce que**
la tête de travail (11) de l'outil (10) est une pointe de préparation qui est de forme tronquée à son extrémité libre.

8. Instrument médical selon la revendication 7,
**caractérisé en ce que**
dans la pointe de préparation, est prévu un dispositif de maintien pour la fixation d'une boucle (6) de l'implant.

9. Instrument médical selon la revendication 8,
**caractérisé en ce que**
le dispositif de maintien est une fente (20) ménagée dans la pointe de préparation.

10. Instrument médical selon une des revendications 1 à 9,
**caractérisé en ce qu'**
à l'extrémité distale du corps (1), est disposé un prolongateur (14) présentant une ouverture traversante (15) qui s'étend dans la direction longitudinale du prolongateur (14) et prolonge le canal (9) qui traverse le corps (1), et à travers laquelle s'étend l'outil (10), le prolongateur (14) et l'ouverture (15) qui s'étend dans ce dernier étant d'une forme incurvée, du moins dans la région qui se raccorde à l'extrémité distale (16) du prolongateur (14).

11. Instrument médical selon la revendication 9,
**caractérisé en ce que**
la courbure est dirigée vers la chambre de réception (2) du corps (1).

12. Instrument médical selon une des revendications 1 à 11,
**caractérisé en ce que**
l'outil médical (10) est une pince de préhension ou une cisaille.

13. Instrument médical selon une des revendications 1 à 11,
**caractérisé en ce que**
l'outil médical (10) est un instrument d'aspiration et/ou de lavage.

14. Instrument médical selon une des revendications 1 à 11,
**caractérisé en ce que**
l'outil médical (10) et un instrument d'électro-chirurgie.

15. Instrument médical selon une des revendications 1 à 11,
**caractérisé en ce que**
l'outil médical (10) est un écarteur.

16. Instrument médical selon une des revendications 1 à 11,
**caractérisé en ce que**
l'outil médical (10) est un instrument à agrafes.

17. Instrument médical selon une des revendications 1 à 16,
**caractérisé en ce que**
la chambre de réception (2) et le canal traversant (9) du corps (10) sont séparés l'un de l'autre par une cloison à l'intérieur du corps (1).
